Europäisches Patentamt

(19) European Patent Office

Office européen des brevets

(11) Publication number: **0 252 705 B1**

(12) # EUROPEAN PATENT SPECIFICATION

(45) Date of publication of patent specification :
23.10.91 Bulletin 91/43

(51) Int. Cl.[5]: **C07C 5/41, C07C 2/84, C07C 15/02, B01J 29/28**

(21) Application number : **87305950.5**

(22) Date of filing : **06.07.87**

(54) Aromatisation of aliphatics over gallium-containing zeolites.

(30) Priority : **07.07.86 US 882875**
**07.07.86 US 882863**

(43) Date of publication of application :
**13.01.88 Bulletin 88/02**

(45) Publication of the grant of the patent :
**23.10.91 Bulletin 91/43**

(84) Designated Contracting States :
**BE DE FR GB IT NL**

(56) References cited :
**EP-A- 0 050 021**
**EP-A- 0 130 251**
**EP-A- 0 202 000**

(56) References cited :
**EP-A- 0 223 345**
**US-A- 4 180 689**
**US-A- 4 350 835**
**US-A- 4 590 323**

(73) Proprietor : **MOBIL OIL CORPORATION**
**3225 Gallows Road**
**Fairfax, Virginia 22037-0001 (US)**

(72) Inventor : **Chu, Cynthia Ting-Wah**
**18 Indian Run Road**
**Princeton Junction, NJ 08550 (US)**

(74) Representative : **Jones, Alan John et al**
**CARPMAELS & RANSFORD 43 Bloomsbury Square**
**London, WC1A 2RA (GB)**

Jouve, 18, rue Saint-Denis, 75001 PARIS

## Description

This invention relates to a process for the conversion of a gaseous paraffinic feed containing a major proportion of $C_2$ to $C_{12}$ aliphatic hydrocarbons to aromatics in the presence of a crystalline zeolite catalyst containing gallium.

US-A-4,180,689 teaches that by using catalysts which contain gallium and which are prepared from specific types of aluminosilicates, improved yields of aromatic hydrocarbons may be obtained if the gallium-containing catalysts are prepared from specific types of aluminosilicates. This patent further discloses that if the gallium is either exchanged for one of the cations or protons or impregnated into the zeolitic cavities, surprisingly high catalytic selectivity is obtained, especially in hydrocarbon conversion processes. The feed for this process is a $C_3$-$C_{12}$ feedstock of either a single component or mixtures of saturated and unsaturated hydrocarbons.

US-A-4,120,910 discloses that aromatic compounds can be produced by contacting, in the absence of added air or oxygen under suitable conversion conditions, a gaseous, paraffinic feed stock containing a high percentage of ethane with a ZSM-5 type crystalline aluminosilicate zeolite catalyst having incorporated therein a minor amount of a metal or metal oxide from Group VIII, IIB, or IB of the Periodic Table. Especially preferred is a zinc-copper mixture.

US-A-4,304,686 teaches the aromatization of aliphatic hydrocarbons utilizing as catalyst a zeolite having an alumina-silica ratio of 10:1 to 500:1 in which at least some of the cations have been exchanged for gallium ions.

US-A-4,350,835 teaches a catalytic process for converting a feedstock comprising a high percentage of ethane to aromatics employing as a catalyst a zeolite with a silica-alumina ratio of at least 12 and having incorporated therein a minor amount of non-framework gallium.

EP-A-50, 021 teaches a process for producing aromatic hydrocarbons by contacting a feedstock containing at least 70% by weight of $C_2$ hydrocarbons with a catalyst comprising an aluminosilicate with a silica-alumina molar ratio of at least 5:1 and in which either gallium is deposited thereon, or cations have been exchanged with gallium ions.

EP-A-130251 discloses a process for aromatizing a $C_2$-$C_{12}$ hydrocarbon feed using as containst a zéolite which is impregnated with zinc and to impregnated with gallium and, optionally, pulladium.

According to the present invention a process for producing aromatic compounds comprises contacting a feed containing at least 50 weight percent of $C_2$ to $C_{12}$ aliphatic hydrocarbons, under conversion conditions, with a catalyst comprising a crystalline zeolite having a constraint index of 1 to 12 and a silica/alumina mole ratio greater than 70, and from 0.5 to 10 weight percent of gallium, said zeolite and said gallium having been brought together after crystallisation of the zeolite. Preferably the zeolite has a silica/alumina mole ratio greater than 200, even more preferably greater than 550.

At least part of said gallium is in tetrahedral coordination in the framework of the zeolite, and in a favoured embodiment the catalyst contains 0.5 to 5% wt. of gallium in tetrahedral coordination and 1 to 10% wt. of total gallium. The gallium may be introduced into tetrahedral coordination in the framework of the zeolite by treatment of the zeolite with a liquid gallium-containing medium under alkaline conditions, notably at a pH greater than 8.

Preferably the zeolite is ZSM-5, ZSM-11, ZSM-12, ZSM-23, ZSM-35, ZSM-38 or ZSM-48, and the catalyst may further comprises a porous matrix with which the zeolite is composited, the zeolite advantageously constituting 5 to 80% wt. of its composite with the matrix. Suitable conversion conditions comprise a temperature of 482 to 760°C, a pressure of 100 to 2860 kPa and a LHSV of 0.1 to 5, preferably a temperature of 593 to 677°C, a pressure of 100 to 790 kPa and a LHSV of 0.3 to 3.

An unexpected advantageous effect of the invention, in comparison with prior art processes dedicated to the aromatisation of similar feedstocks, is the obtaining of a high product benzene/toluene ratio, typically greater than 1:1 (weight basis).

The process of the present invention is carried out using catalysts in which gallium-comprising metal is impregnated on the surface of the zeolite, optionally concurrently with ion-exchange, in addition to being incorporated under alkaline conditions into the framework of the zeolite. Any alkaline material can be used for this purpose which is compatible with the gallium-comprising metal reagent, e.g. an aqueous solution of alkali metal hydroxides such as sodium hydroxide with a concentration in the range, for example of about 0.1 to 1 N. In carrying out the insertion of the gallium-comprising metal by this method, the zeolite may be refluxed in a solution of alkaline material and gallium-comprising metal reagent, e.g. any of various water-soluble compounds such as sulfates and nitrates, ammonia-exchanged to remove or reduce alkali metal cations, and calcined to obtain the final catalyst. Suitable refluxing conditions comprise a temperature of 50 to 100°C for a period of 0.5 to 20 hours.

Where the catalyst composition is prepared by using a compound of gallium-comprising metal which ionizes in aqueous solution, for example gallium nitrate, some of the gallium ions are generally exchanged with the cations in the zeolite even if the preparation was directed to impregnation of or insertion into the zeolite.

Whichever method of catalyst preparation is used, the amount of gallium present in the catalyst compositions, (metal plus zeolite), may vary for instance between 0.1 and 15, preferably between 0.5 and 10, percent by weight. Of the total amount of added metal, gallium may comprise an amount from over 50 to 100 wt.%. The portion of the added metal which is not gallium may be any of various suitable metals in Groups I through VIII of the Periodic Table including by way of example, zinc, platinum, rhenium, cobalt, titanium, tellurium, sodium, nickel, boron, chromium, aluminum, copper, platinum, calcium and rare earth metals.

The gallium in the catalyst may therefore be entirely in tetrahedral coordination in the framework of the zeolite, or partly framework and partly non-framework. Thus in an embodiment previously mentioned, a favoured catalyst formulation contains 1 to 10% wt. total gallium of which half is framework gallium. Certain of the aforementioned metals which may be co-present with gallium may similarly assume framework and non-framework roles.

Although the zeolites utilized in the process have extremely low alumina contents, i.e, silica to alumina mole ratios exceeding 550, they are nevertheless very active. The activity is surprising since catalytic activity is generally attributed to framework aluminum atoms and/or cations associated with these aluminum atoms. These zeolites retain their crystallinity for long periods in spite of the presence of steam at high temperature which induces irreversible collapse of the framework of other zeolites, e.g. of the X and A type. Furthermore, carbonaceous deposits, when formed, may be removed by burning at higher than usual temperatures to restore activity. These zeolites, used as catalysts, generally have low coke-forming activity and therefore are conducive to long times on stream between regenerations by burning carbonaceous deposits with oxygen-containing gas such as air.

The significance and manner of determination of constraint index are described in our GB-A-1,446,522. We do however draw attention to the fact that testing parameters to some degree determine the precise value of constraint index yielded for a given zeolite by an individual test, and that any zeolite which is capable, upon testing within the prescribed conditions, of manifesting a constraint index of 1 to 12 is considered for the purposes of the present specification to be a zeolite having a constraint index of 1 to 12.

Constraint Index (CI) values for some typical materials are:

| | CI (at test temperatures - °C) | |
|---|---|---|
| ZSM-4 | 0.5 | (316) |
| ZSM-5 | 6-8.3 | (371-316) |
| ZSM-11 | 5-8.7 | (371-316) |
| ZSM-12 | 2.3 | (316) |
| ZSM-20 | 0.5 | (371) |
| ZSM-22 | 7.3 | (427) |
| ZSM-23 | 9.1 | (427) |
| ZSM-34 | 50 | (371) |
| ZSM-35 | 4.5 | (454) |
| ZSM-38 | 2 | (510) |
| ZSM-48 | 3.5 | (538) |
| ZSM-50 | 2.1 | (427) |
| TMA Offretite | 3.7 | (316) |
| TEA Mordenite | 0.4 | (316) |
| Clinoptilolite | 3.4 | (510) |
| Mordenite | 0.5 | (316) |
| REY | 0.4 | (316) |
| Amorphous Silica-alumina | 0.6 | (538) |
| Dealuminized Y | 0.5 | (510) |
| Erionite | 38 | (316) |
| Zeolite Beta | 0.6-2.0 | (316-399) |

Zeolites ZSM-5, ZSM-11, ZSM-12, ZSM-23, ZMS-35, ZSM-38 and ZSM-48 are defined by the x-ray data set forth in US-A-3,702,886, 3,709,979, 3,832,449, 4,076,842, 4,016,245, 4,046,859 and 4,350,835 respectively.

The specific zeolites described, when prepared in the presence of organic cations, are substantially catalytically inactive, possibly because the intra-crystalline free space is occupied by organic cations from the forming solution. They may be activated by heating in an inert atmosphere at about 540°C for one hour, for example, followed by base exchange with ammonium salts followed by calcination at about 540°C in air. The presence of organic cations in the forming solution may not be absolutely essential to their formation but does appear to favor it. In many cases it is desirable to activate the zeolite by base exchange with ammonium salts followed by calcination in air at about 540°C for from about 15 minutes to about 24 hours.

Natural zeolites may sometimes be converted to zeolite structures of the class herein identified by various activation procedures and other treatments such as base exchange, steaming, alumina extraction and calcination, alone or in combinations. Natural minerals which may be so treated include ferrierite, brewsterite, stilbite, dachiardite, epistilbite, heulandite and clinoptilolite.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with gallium and, if desired other suitable metal cations, of Groups I through VII of the Periodic Table as mentioned previously.

It may be useful to incorporate the above-mentioned crystalline zeolite into a matrix comprising another material resistant to the temperature and other conditions employed in the process to be catalysed. Such matrix material is useful as a binder and imparts greater resistance to the catalyst for the severe temperature, pressure and reactant feed stream velocity conditions encountered in many conversion processes. Useful matrix materials include both synthetic and naturally occurring substances, as well as inorganic materials such as clay, silica and/or metal oxides, and are fully described in our EP-A-1695.

The feed stream to the process of this invention contains at least 50% by weight of at least one aliphatic hydrocarbon containing 2 to 12 carbon atoms. The hydrocarbon may be straight chain, open chain or cyclic and may be saturated or unsaturated. Some contemplated hydrocarbons are ethane, propane, propylene, n-butane, n-butenes, isobutane, isobutene, straight-and branch chain hexanes, hexenes, octanes, octenes, decanes and decenes, cyclohexane and cyclohexene. The effluent from the reaction zone is separated and distilled to remove the desired aromatic product and the remainder may be recycled for further reaction.

The following examples further illustrate the invention. Examples 1 to 3 are embodiments of processes carried out within the invention, whilst Comparative Examples A to C show the results obtained when at least one operating parameter is outside the scope of the invention.

### EXAMPLE 1

This example illustrates the preparation and use of a gallium–containing zeolite catalyst in which some of the gallium is inserted into the framework of a zeolite under alkaline conditions.

Ten grams of HZSM-5 having a silica-alumina ratio of 880 were refluxed with 2.5 grams of $Ga_2(SO_4)_3.18H_2O$ in a solution of 0.2 N NaOH for two hours. The catalyst was then ammonia-exchanged and calcined at 538°C in air for 4 hours. The final catalyst contained 4.0 wt.% of added gallium, of which 29% was determined by the ammonia TPD method to be incorporated within the framework of the zeolite crystals, and 0.36 wt.% of $Al_2O_3$.

This catalyst was tested for aromatization activity by loading it into a reactor and introducing n-hexane at a temperature of 538°C (1000°F), atmospheric pressure, and an LHSV of 0.59. After various times on stream, the product was analyzed. Hydrogen and light gases ($C_1$-$C_3$) were analyzed by a refinery gas analysis GC, $C_3$+ gases were analyzed on a n-octane-porasil C column, and liquid products were analyzed on a DB-1 Durabond capillary column. The results are shown in Table 1, where the numbers all signify weight percent of product except as indicated, "$C_1+C_2$" is the total of methane and ethane, "$C_2$=+" is the total of ethylene and higher aliphatics and "Aromatics Selectivity" is defined as follows.

Aromatics yield (wt.%)

$$C_1 + C_2 \ (wt.\%) \ + \ \text{aromatics yield (wt.\%)}$$

### EXAMPLE 2

The procedure of Example 1 was followed except that the catalyst preparation solution contained 15 grams of the ZSM-5 which was refluxed with 3.75 grams of $Ga_2(SO_4)_3.18H_2O$. The finished catalyst contained 7.3 wt.% of gallium, 65% of which was in the framework of the zeolite, and 0.39 wt.% of $Al_2O_3$. The results are shown in Table 1.

### COMPARATIVE EXAMPLE A

This example illustrates the preparation and use of a catalyst in which gallium is deposited in a zeolite substrate without entering the framework.

15 grams of $Ga(NO_3)_3.xH_2O$ were dissolved in $H_2O$ and impregnated on 7.5 grams of a ZSM-5 zeolite with the same silica-alumina ratio as Examples 1 and 2, viz. 880. The catalyst was then calcined in air at 538°C. The finished catalyst contained 10.2 wt.% of deposited gallium. The catalyst was then tested for aromatization activity as described in Example 1. The results are shown in Table 1.

Table 1

| Example | 1 | | | 2 | | | | A |
|---|---|---|---|---|---|---|---|---|
| Time on Stream(hrs.) | 7 | 12 | 18 | 6 | 12 | 17 | .7 | 30 |
| $H_2$ | 1.19 | 2.87 | 1.91 | 3.08 | 2.35 | 2.31 | 1.86 | 0.96 |
| Methane | 2.74 | 3.82 | 5.85 | 3.82 | 4.41 | 4.92 | 0.01 | 0.74 |
| Ethane | 4.62 | 8.24 | 6.07 | 9.70 | 7.50 | 7.48 | 4.82 | 1.56 |
| Propane | 9.41 | 9.56 | 13.24 | 10.04 | 10.82 | 13.76 | 2.50 | 1.75 |
| n-Butane | 3.64 | 3.08 | 3.92 | 2.32 | 2.80 | 3.44 | 0.74 | 0.88 |
| i-Butane | 5.21 | 5.34 | 5.65 | 3.08 | 3.60 | 5.85 | 1.70 | 2.60 |
| n-Pentane | 0.04 | 0.02 | 0.06 | 0.04 | 0.05 | 0.02 | 0.08 | 0.08 |
| i-Pentane | 1.98 | 0.72 | 0.10 | 0.04 | 0.15 | 0.03 | 0.73 | 0.95 |
| n-Hexane | 15.57 | 15.02 | 12.85 | 4.62 | 8.00 | 10.17 | 56.83 | 70.39 |
| $C_6$+Aliphatics | 3.64 | 0.72 | 0.73 | 6.09 | 4.46 | 0.10 | 0.80 | 1.07 |
| Ethylene | 2.82 | 6.26 | 4.12 | 3.53 | 2.88 | 3.58 | 3.79 | 2.31 |
| Propylene | 9.70 | 10.81 | 12.05 | 5.84 | 7.34 | 10.82 | 5.72 | 3.74 |
| $C_4$ Olefins | 5.10 | 5.35 | 2.21 | 0.24 | 1.19 | 1.82 | 4.11 | 4.72 |
| $C_5$ Olefins | 2.77 | 0.62 | 0.39 | 2.14 | 0.36 | 0.15 | 0.55 | 0.61 |
| Benzene | 14.56 | 11.44 | 13.30 | 17.32 | 17.68 | 12.78 | 4.32 | 2.89 |
| Toluene | 9.08 | 7.47 | 8.60 | 13.15 | 12.03 | 7.97 | 3.69 | 1.79 |
| $C_8$ Aromatics | 5.30 | 6.67 | 6.41 | 10.09 | 9.59 | 8.50 | 4.45 | 1.81 |
| $C_9$ Aromatics | 1.39 | 1.77 | 2.03 | 2.19 | 2.44 | 2.41 | 1.61 | 0.86 |
| $C_{10}$ Aromatics | 0.90 | 0.25 | 0.50 | 1.50 | 1.35 | 1.22 | 0.41 | 0.21 |
| $C_{11}$ Aromatics | 0.35 | -- | 0.03 | 1.17 | 1.12 | 0.91 | 0.24 | 0.07 |
| Material Balance (% recovered) | | | | | | | | |
| C | 106 | 95 | 103 | 100 | 100 | 100 | 95 | 98 |
| H | 97 | 99 | 102 | 99 | 96 | 99 | 98 | 101 |
| $C_1 + C_2$ | 7.45 | 12.41 | 12.15 | 13.95 | 12.20 | 12.70 | 5.94 | 2.33 |
| Aromatics | 31.96 | 28.42 | 31.47 | 46.86 | 45.27 | 36.39 | 15.00 | 7.72 |
| $C_2$=+ | 60.59 | 59.17 | 56.38 | 39.19 | 42.53 | 50.91 | 79.06 | 89.95 |
| Aromatics Selectivity | 81.10 | 69.61 | 72.15 | 77.06 | 78.77 | 74.13 | 71.63 | 76.82 |

The aromatization activity of the catalysts of Examples 1, 2 and A as indicated by the results of Table 1 show a relatively high aromatics selectivity and a concomitant low production of $C_1+C_2$ which are considered particularly undesirable by-products of aromatics production. However, comparison of the results of Examples 1 and 2 wherein the catalyst contained a substantial proportion of gallium inserted into the framework of the gallium under alkaline conditions, with those of Example A wherein the gallium was deposited on the zeolite by ion exchange, shows that higher aromatics selectivites are obtained after a time on stream of 7 hours or less with catalysts containing some inserted gallium than those containing all exchanged gallium.

COMPARATIVE EXAMPLE B

This example illustrates the preparation and use of a catalyst utilizing a zeolite in which the silica-alumina ratio is lower than that called for by the invention.

The procedure of Example A was followed except that the silica-alumina ratio of the ZSM-5 was 70. The finished catalyst contained 6.8 wt.% of deposited gallium. The results of aromatization of n-hexane with this catalyst are shown in Table 2.

COMPARATIVE EXAMPLE C

This example illustrates the preparation and use of a catalyst in which the silica-alumina ratio is lower than that called for by the invention and which contains gallium deposited by impregnation.

2 grams of $Ga(NO_3)_3.xH_2O$ were dissolved in 10cc of water and 5 grams of HZSM-5 with a silica-alumina ratio of 40 were added to the solution. The mixture was dried overnight and calcined at 538°C for 6 hours. The finished catalyst contained 7.3 wt.% of impregnated gallium. Use of this catalyst for aromatization of n-hexane as described in Example 1 yielded the results shown in Table 2.

COMPARATIVE EXAMPLE D

This example illustrates the use of a catalyst consisting of a zeolite having a lower silica-alumina ratio than is defined by the invention and no added metal.

n-Hexane was subjected to aromatization using the conditions of Example 1 and a catalyst consisting of HZSM-5 with a silica-alumina ratio of 70 and no added metal such as gallium. The results obtained are shown in Table 2.

EP 0 252 705 B1

## Table 2

| Example | | | B | | | | C | | D |
|---|---|---|---|---|---|---|---|---|---|
| Time on Stream | 7.5 | 14.5 | 21.5 | 43.5 | 60 | 7.5 | 15 | 31.5 | 5.5 |
| $H_2$ | 4.68 | 4.05 | 3.70 | 3.90 | 3.62 | 4.29 | 3.68 | 3.69 | 0.23 |
| Methane | 6.31 | 5.22 | 4.89 | 4.84 | 4.73 | 8.94 | 7.67 | 5.52 | 8.15 |
| Ethane | 21.44 | 18.10 | 15.46 | 16.91 | 16.52 | 22.88 | 19.63 | 21.36 | 14.99 |
| Propane | 18.71 | 17.18 | 15.45 | 15.33 | 15.14 | 8.21 | 12.31 | 13.79 | 36.94 |
| n-Butane | 2.11 | 3.01 | 3.25 | 3.20 | 3.01 | 0.21 | 0.43 | 1.97 | 1.54 |
| i-Butane | 1.04 | 2.25 | 3.00 | 3.60 | 3.08 | 0.18 | 0.25 | 1.11 | 1.80 |
| n-Pentane | 0.02 | 0.01 | 0.02 | 0.04 | 0.02 | 0.00 | 0.00 | 0.04 | 0.05 |
| i-Pentane | 0.01 | 0.18 | 0.42 | 0.45 | 0.29 | 0.03 | 0.05 | 0.26 | 0.10 |
| n-Hexane | 0.56 | 0.62 | 1.82 | 2.37 | 5.04 | 0.06 | 0.06 | 0.16 | 0.23 |
| $C_6^+$ Aliphatics | 0.00 | 0.07 | 0.04 | 0.04 | 0.13 | 0.00 | 0.04 | 0.01 | 2.66 |
| Ethylene | 1.75 | 2.98 | 3.80 | 4.56 | 3.86 | 0.64 | 0.55 | 1.99 | 2.76 |
| Propylene | 2.28 | 3.89 | 5.34 | 6.20 | 5.25 | 0.72 | 1.09 | 2.46 | 3.04 |
| $C_4$ Olefins | 0.92 | 1.81 | 2.68 | 3.13 | 2.92 | 0.07 | 0.19 | 0.89 | 1.07 |
| $C_5$ Olefins | 0.01 | 0.03 | 0.14 | 0.18 | 0.21 | 0.01 | 0.04 | 0.04 | 0.85 |
| Benzene | 13.03 | 14.11 | 12.06 | 10.92 | 11.39 | 15.97 | 16.35 | 13.16 | 4.14 |
| Toluene | 13.63 | 13.31 | 12.74 | 10.80 | 10.05 | 19.91 | 19.07 | 15.81 | 8.67 |
| $C_8$ Aromatics | 8.68 | 8.79 | 9.79 | 8.54 | 9.10 | 9.48 | 10.83 | 10.77 | 6.06 |
| $C_9$ Aromatics | 1.44 | 1.59 | 2.78 | 2.56 | 3.44 | 1.33 | 1.66 | 2.02 | 1.43 |
| $C_{10}$ Aromatics | 2.17 | 1.62 | 1.33 | 1.10 | 1.10 | 2.36 | 2.94 | 2.67 | 1.89 |
| $C_{11}$ Aromatics | 1.21 | 1.17 | 1.31 | 1.33 | 1.11 | 3.70 | 3.16 | 2.28 | 3.41 |
| $C_1 + C_2$ | 29.11 | 24.30 | 21.13 | 22.63 | 22.05 | 33.25 | 28.34 | 27.91 | 23.19 |
| Aromatics | 42.14 | 42.30 | 41.55 | 36.68 | 37.54 | 56.16 | 56.07 | 48.50 | 25.66 |
| $C_2=+$ | 28.75 | 33.40 | 37.32 | 40.69 | 40.41 | 10.59 | 15.59 | 23.59 | 51.15 |
| Aromatics Selectivity | 59.14 | 63.51 | 66.29 | 61.84 | 63.00 | 62.81 | 66.43 | 63.47 | 52.53 |

Comparison of the results of the Examples in accordance with the invention with those of Comparative Examples B to D wherein at least one parameter defined by the invention was not employed, shows that substantially higher aromatics selectivities and lower production of $C_1+C_2$ were obtained with the examples under the invention than with the comparative examples.

It will have been noted that use of a zeolite catalyst containing zeolitic gallium in tetrahedral coordination therein in an aromatization process in many cases yields a product in which the ratio of aromatics to the total of methane and ethane ($C_1+C_2$) is higher than that obtained under equivalent conditions but using a zeolite in which all the gallium is ion-exchanged and/or impregnated. This is significant since methane does not usually yield any aromatics or aromatics precursors on recycling, while ethane is more difficult to aromatize than higher alkanes.

## Claims

1. A process for producing aromatic compounds which comprises contacting a feed containing at least 50 weight percent of $C_2$ to $C_{12}$ aliphatic hydrocarbons, under conversion conditions, with a catalyst comprising a crystalline zeolite having a constraint index of 1 to 12 and a silica/alumina mole ratio greater than 70, and from 0.5 to 10 weight percent of gallium, said zeolite and said gallium having been brought together after crystallisation of the zeolite, at least part of said gallium being in tetrahedral coordination in the framework of the zeolite.

2. A process according to claim 1 where in the zeolite has a silica/alumina mole ratio greater than 200.

3. A process according to claim 1 or claim 2 wherein the zeolite has a silica/alumina mole ratio greater than 550.

4. A process according to any preceding claim wherein the catalyst contains 0.5 to 5% wt. of said gallium in tetrahedral coordination and 1 to 10% wt. of total gallium.

5. A process according to any preceding claim wherein gallium assumes tetrahedral coordination in the framework of the zeolite by treatment of the zeolite with a liquid gallium-containing medium under alkaline conditions.

6. A process according to claim 5 wherein the pH of said treatment is at least 8.

7. A process according to any preceding claim wherein the zeolite is ZSM-5, ZSM-11, ZSM-12, ZSM-23, ZSM-35, ZSM-38 or ZSM-48.

8. A process according to any preceding claim wherein the catalyst further comprises a porous matrix with which the zeolite is composited.

9. A process according to claim 8 wherein the zeolite constitutes 5 to 80% wt. of its composite with the matrix.

10. A process according to any preceding claim wherein the conversion conditions comprise a temperature of 482 to 760°C a pressure of 100 to 2860 kPa and a LHSV of 0.1 to 5.

11. A process according to any preceding claim wherein conversion conditions comprise a temperature of 593 to 677°C, a pressure of 100 to 790 kPa and a LHSV of 0.3 to 3.

## Patentansprüche

1. Verfahren zur Herstellung aromatischer Verbindungen, das den Kontakt einer Zufuhr, die mindestens 50 Gew.-% aliphatischer $C_2$ bis $C_{12}$-Kohlenwasserstoffe enthält, bei Umwandlungsbedingungen mit einem Katalysator umfaßt, der einen kristallinen Zeolith mit einem Zwangsindex von 1 bis 12 und ein Silicumdioxid/Aluminiumoxid-Molverhältnis von mehr als 70 aufweist und von 0,5 bis 10 Gew.-% Gallium umfaßt, wobei der Zeolith und das Gallium nach der Kristallisation des Zeoliths zusammengebracht wurden, wobei zumindest ein Teil des Galliums im Gitter des Zeoliths in tetraedrischer Koordinierung vorliegt.

2. Verfahren nach Anspruch 1, worin der Zeolith ein Siliciumdioxid/Aluminiumoxid-Molverhältnis von mehr als 200 aufweist.

3. Verfahren nach Anspruch 1 oder 2, worin der Zeolith ein Siliciumdioxid/Aluminiumoxid-Molverhältnnis von mehr als 550 aufweist.

4. Verfahren nach einem der vorstehenden Ansprüche, worin der Katalysator 0,5 bis 5 Gew.-% des Galliums in tetraedrischer Koordinierung und 1 bis 10 Gew.-% des gesamten Galliums enthält.

5. Verfahren nach einem der vorstehenden Ansprüche, worin das Gallium durch Behandlung des Zeoliths mit einem flüssigen Gallium enthaltenden Medium bei alkalischen Bedingungen die tetraedrische Koordinierung im Gitter des Zeoliths einnimmt.

6. Verfahren nach Anspruch 5, worin der pH-Wert dieser Behandlung mindestens 8 beträgt.

7. Verfahren nach einem der vorstehenden Ansprüche, worin der Zeolith ZSM-5, ZSM-11, ZSM-12, ZSM-23, ZSM-35, ZSM-38 oder ZSM-48 ist.

8. Verfahren nach einem der vorstehenden Ansprüche, worin der Katalysator außerdem eine poröse Matrix umfaßt, mit der der Zeolith zusammengesetzt ist.

9. Verfahren nach Anspruch 8, worin der Zeolith 5 bis 80 Gew.-% der Zusammensetzung mit der Matrix bildet.

10. Verfahren nach einem der vorstehenden Ansprüche, worin die Umwandlungsbedingungen eine Temperatur von 482 bis 760°C, einen Druck von 100 bis 2860 kPa und eine LHSV von 0,1 bis 5 umfassen.

11. Verfahren nach einem der vorstehenden Ansprüche, worin die Umwandlungsbedingungen eine Temperatur von 593 bis 677°C, einen Druck von 100 bis 790 kPa und eine LHSV von 0,3 bis 3 umfassen.


## Revendications

1. Un procédé de production de composés aromatiques qui comprend la mise en contact d'une charge contenant au moins 50% en poids d'hydrocarbures aliphatiques en $C_2$ à $C_{12}$, dans des conditions de conversion, avec un catalyseur comprenant une zéolite cristalline ayant un indice de contrainte de 1 à 12 et un rapport molaire silice/alumine supérieur à 70, et de 0,5 à 10% en poids de gallium, ladite zéolite et ledit gallium ayant été mis en contact intime après la cristallisation de la zéolite, et une part au moins dudit gallium étant présent aux sites de coordination tétraédrique dans la structure de la zéolite.

2. Un procédé selon la revendication 1, dans lequel la zéolite a un rapport molaire silice/alumine supérieur à 200.

3. Un procédé selon la revendication 1 ou la revendication 2, dans lequel la zéolite a un rapport molaire silice/alumine supérieur à 550.

4. Un procédé selon l'une quelconque des revendications précédentes, dans lequel le catalyseur contient de 0,5 à 5% en poids dudit gallium au niveau des sites de coordination tétraédrique et de 1 à 10% en poids de gallium total.

5. Un procédé selon l'une quelconque des revendications précédentes, dans lequel le gallium occupe les sites de coordination tétraédrique dans la structure de la zéolite par traitement de la zéolite avec un milieu liquide contenant du gallium dans des conditions alcalines.

6. Un procédé selon la revendication 5, dans lequel le pH du traitement est d'au moins 8.

7. Un procédé selon l'une quelconque des revendications précédentes, dans lequel la zéolite est du type ZSM-5, ZSM-11, ZSM-12, ZSM-23, ZSM-35, ZSM-38 ou ZSM-48.

8. Un procédé selon l'une quelconque des revendications précédentes, dans lequel le catalyseur comprend de plus une matrice poreuse avec laquelle la zéolite est mélangée.

9. Un procédé selon la revendication 8, dans lequel la zéolite constitue de 5 à 80% en poids de son composite avec la matrice.

10. Un procédé selon l'une quelconque des revendications précédentes, dans lequel les conditions de conversion comprennent une température de 482 à 760°C, une pression de 100 à 2 860 kPa et une vitesse spatiale horaire liquide (VSHL) de 0,1 à 5.

11. Un procédé selon l'une quelconque des revendications précédentes, dans lequel les conditions de conversion comprennent une température de 593 à 677°C, une pression de 100 à 790 kPa et une vitesse spatiale horaire liquide (VSHL) de 0,3 à 3.